# EUROPÄISCHE PATENTANMELDUNG

(11) **EP 2 317 320 A2**
(43) Veröffentlichungstag der Anmeldung: **04.05.2011**
(21) Anmeldenummer: 10183096.6
(22) Anmeldetag: 06.04.2006
(51) Int. Cl.: G01N 33/574, G01N 33/68, C07K 7/08

(54) **Polypeptidmarker zur Diagnose von Prostatakrebs**

(30) Priorität: 07.04.2005 EP 05102741; 21.06.2005 EP 05105434
(62) Teilanmeldung aus: 06725599.2
(71) Anmelder: mosaiques diagnostics and therapeutics AG, 30625 Hannover (DE)
(72) Erfinder: Mischak, Harald, 31319 Sehnde (DE)
(74) Vertreter: von Kreisler Selting Werner

(57) **Zusammenfassung**

Verfahren zur Diagnose von Prostatakrebs umfassend den Schritt der Bestimmung einer An- oder Abwesenheit mindestens dreier Polypeptidmarkers in einer Probe, wobei der Polypeptidmarker ausgewählt ist aus den Markern 1 bis 44 und 52 bis 78 (Frequenzmarker), oder der Bestimmung der Amplitude mindestens eines Polypeptidmarkers, ausgewählt aus den Markern 45 bis 51 und 79-115 (Amplitudenmarker), die durch folgende Werte für die Molekularmassen und die Migrationszeit charakterisiert sind:

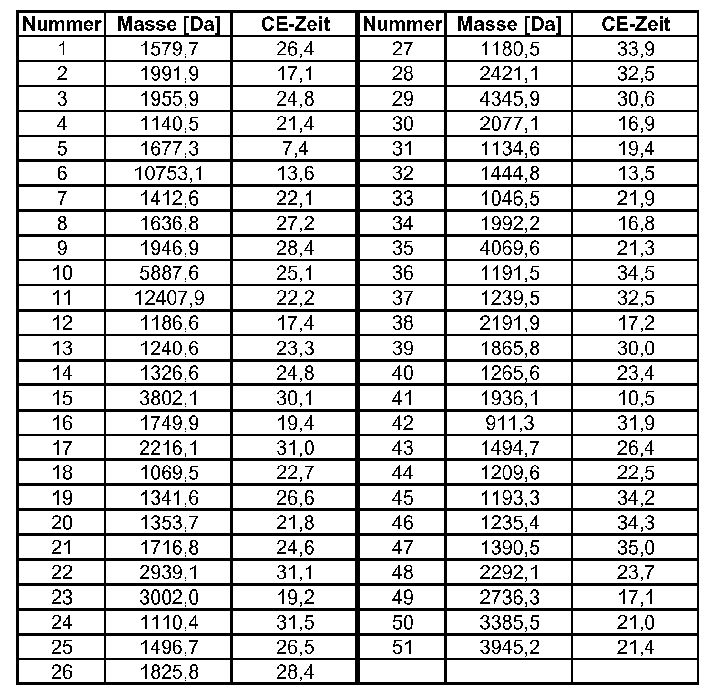

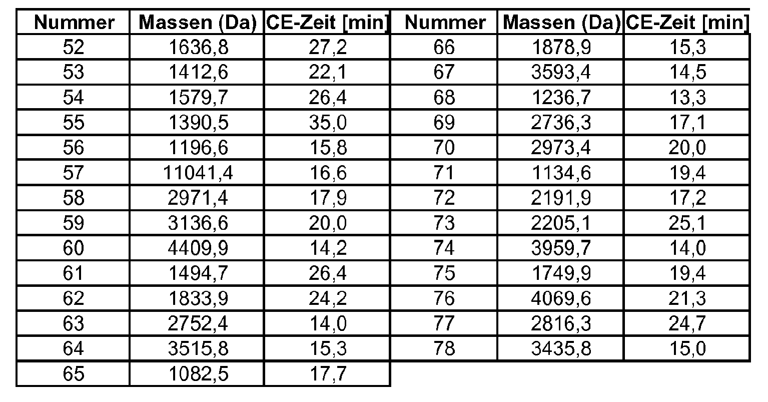

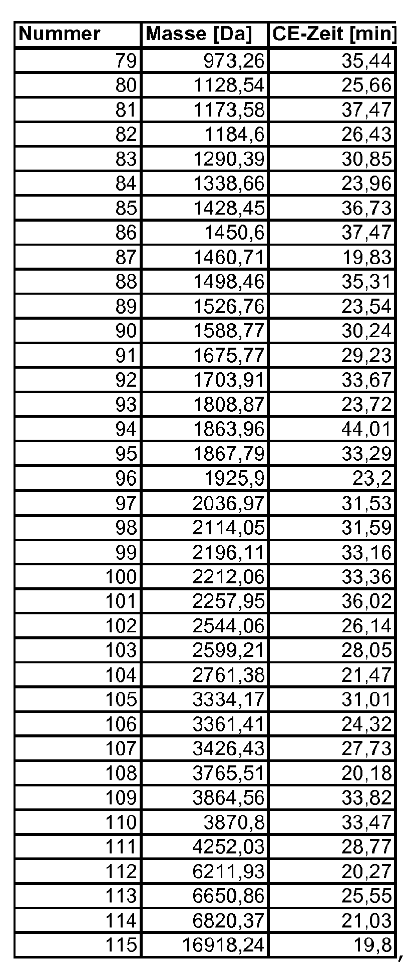

wobei es sich bei der Probe um eine Urinprobe oder Samenflüssigkeitsprobe handelt.

## Beschreibung

Die vorliegende Erfindung betrifft die Verwendung der An- oder Abwesenheit eines oder mehrerer Peptidmarker in einer Probe eines Individuums zur Diagnose von Prostatakrebs (PCA) sowie ein Verfahren zur Diagnose von Prostatakrebs, wobei die An- oder Abwesenheit des oder der Peptidmarker(s) indikativ für das Vorliegen von Prostatakrebs ist.

Das Karzinom der Prostata (Vorsteherdrüse) ist eine der häufigsten Krebserkrankungen beim Mann. Da es erst im Stadium der fortgeschrittenen Erkrankung zu Beschwerden kommt, kann der Krebs nur durch regelmäßige Früherkennungsuntersuchungen (Tastbefund und PSA-Wert (Prostate specific antigen) im Blut) im Frühstadium diagnostiziert werden. Zur Sicherung der Verdachtsdiagnose wird mittels Feinnadelbiopsie eine Gewebsprobe entnommen.

Für die Therapie stehen mehrere Möglichkeiten zur Verfügung, die sich nach der Art und dem Stadium des Tumors sowie nach den individuellen Bedürfnissen des Patienten richten: Im frühen Stadium stehen die Seed-Implantation (minimal-invasive Einbringung von radioaktiven Jod 125- Strahlern in die Prostata) oder die operative Entfernung des Tumors und Bestrahlung von außen zur Verfügung.

Zum Zeitpunkt der Diagnosestellung hat bei einem Drittel der Patienten bereits eine Metastasierung in andere Organe stattgefunden; die Erkrankung ist zu diesem Zeitpunkt bereits kaum noch heilbar. Durch Strahlen-, Chemo- oder Hormontherapie (Kombinationen sind möglich) kann jedoch die weitere Ausbreitung des Krebses verzögert werden. Bei isoliertem Befall der Prostata, also wenn noch keine Absiedlung stattgefunden hat, ist die Prognose jedoch günstig.

Viel häufiger als das Karzinom tritt ein gutartiger Tumor (Adenom) der Prostata auf, die benigne Prostatahyperplasie (BPH). Nach dem Stand der Technik kann sie nur sehr unzuverlässig über den PSA-Wert von einem bösartigen Karzinom unterschieden werden. Auch hier muss eine Biopsie erfolgen, um eine klare Diagnose treffen zu können.

Wie bereits beschrieben, gibt es keine nichtinvasive und verlässliche Früherkennung des Prostatakrebses. Eine klare Diagnose ist bisher mit invasiven Eingriffen wie der Biopsie verbunden. Es stellte sich also die Aufgabe, ein Verfahren und eine Methode zur möglichst wenig invasiven, schnellen und kostengünstigen Diagnose des Prostatakrebses zu finden.

WO 03/027710 beschreibt Proteinbiomarker zur Unterscheidung von Prostatakarzinomzellen und BPH. In der Anmeldung werden Marker beschrieben, die große Unschärfen aufweisen (im Bereich von mehr als ± 0,5%). Aus der Vielzahl von Molekülen in diesem Bereich ist aufgrund der ungenauen Angaben eine Zuordnung zu einzelnen Markern praktisch nicht möglich. Als Probenmaterial wird bevorzugt ein Zelllysat aus Prostataepithelzellen eingesetzt, was eine aufwendige Probenentnahme erfordert. Aufgrund der Vielzahl der von der Definition umfassten Proteine und der Schwierigkeit der Probenentnahme ist das Verfahren wenig geeignet.

WO 01/25791 beschreibt Verfahren zur Diagnose von Prostatakarzinomen unter Verwendung von Markern. Die genannten Marker entstammen dem Protein Semenogelin I. Die angegebenen Massen sind nur mit einer Genauigkeit von ± 0,5% bestimmt. Untersuchungen zeigen, dass die genannten Marker ungeeignet sind.

Überraschender Weise wurde nun gefunden, das bestimmte Peptidmarker in einer Probe eines Individuums zur Diagnose von Prostatakrebs und zur Differentialdiagnose zur Unterscheidung von Prostatakrebs und benigner Prostatahyperplasie (BPH) verwendet werden können. Insbesondere können die Proben Urin- oder Samenflüssigkeitsproben sein, die nicht-invasiv entnommen werden.

Folglich ist ein Gegenstand der vorliegenden Erfindung die Verwendung der An- oder Abwesenheit mindestens drei Polypeptidmarker in einer Probe eines Individuums zur Diagnose von Prostatakrebs, wobei der Polypeptidmarker ausgewählt ist aus den Polypeptidmarkern Nr. 1 bis 115, die durch die die in Tabelle 1 angegebenen Molekularmassen und ihre Migrationszeiten charakterisiert sind.

**Tabelle 1: Polypeptidmarker zur Diagnose von Prostatakrebs (PCA) sowie ihre Molekularmassen und Migrationszeiten (CE-Zeit in Minuten):**

| **Nummer** | **Masse [Da]** | **CE-Zeit** | **Nummer** | **Masse [Da]** | **CE-Zeit** |
|---|---|---|---|---|---|
| 1 | 1579,7 | 26,4 | 27 | 1180,5 | 33,9 |
| 2 | 1991,9 | 17,1 | 28 | 2421,1 | 32,5 |
| 3 | 1955,9 | 24,8 | 29 | 4345,9 | 30,6 |
| 4 | 1140,5 | 21,4 | 30 | 2077,1 | 16,9 |
| 5 | 1677,3 | 7,4 | 31 | 1134,6 | 19,4 |
| 6 | 10753,1 | 13,6 | 32 | 1444,8 | 13,5 |
| 7 | 1412,6 | 22,1 | 33 | 1046,5 | 21,9 |
| 8 | 1636,8 | 27,2 | 34 | 1992,2 | 16,8 |
| 9 | 1946,9 | 28,4 | 35 | 4069,6 | 21,3 |
| 10 | 5887,6 | 25,1 | 36 | 1191,5 | 34,5 |
| 11 | 12407,9 | 22,2 | 37 | 1239,5 | 32,5 |
| 12 | 1186,6 | 17,4 | 38 | 2191,9 | 17,2 |
| 13 | 1240,6 | 23,3 | 39 | 1865,8 | 30,0 |
| 14 | 1326,6 | 24,8 | 40 | 1265,6 | 23,4 |
| 15 | 3802,1 | 30,1 | 41 | 1936,1 | 10,5 |
| 16 | 1749,9 | 19,4 | 42 | 911,3 | 31,9 |
| 17 | 2216,1 | 31,0 | 43 | 1494,7 | 26,4 |
| 18 | 1069,5 | 22,7 | 44 | 1209,6 | 22,5 |
| 19 | 1341,6 | 26,6 | 45 | 1193,3 | 34,2 |
| 20 | 1353,7 | 21,8 | 46 | 1235,4 | 34,3 |
| 21 | 1716,8 | 24,6 | 47 | 1390,5 | 35,0 |
| 22 | 2939,1 | 31,1 | 48 | 2292,1 | 23,7 |
| 23 | 3002,0 | 19,2 | 49 | 2736,3 | 17,1 |
| 24 | 1110,4 | 31,5 | 50 | 3385,5 | 21,0 |
| 25 | 1496,7 | 26,5 | 51 | 3945,2 | 21,4 |
| 26 | 1825,8 | 28,4 | | | |
| | | | | | |

| **Nummer** | **Massen (Da)** | **CE-Zeit [min]** | **Nummer** | **Massen (Da)** | **CE-Zeit [min]** |
|---|---|---|---|---|---|
| 52 | 1636,8 | 27,2 | 66 | 1878,9 | 15,3 |
| 53 | 1412,6 | 22,1 | 67 | 3593,4 | 14,5 |
| 54 | 1579,7 | 26,4 | 68 | 1236,7 | 13,3 |
| 55 | 1390,5 | 35,0 | 69 | 2736,3 | 17,1 |
| 56 | 1196,6 | 15,8 | 70 | 2973,4 | 20,0 |
| 57 | 11041,4 | 16,6 | 71 | 1134,6 | 19,4 |
| 58 | 2971,4 | 17,9 | 72 | 2191,9 | 17,2 |
| 59 | 3136,6 | 20,0 | 73 | 2205,1 | 25,1 |
| 60 | 4409,9 | 14,2 | 74 | 3959,7 | 14,0 |
| 61 | 1494,7 | 26,4 | 75 | 1749,9 | 19,4 |
| 62 | 1833,9 | 24,2 | 76 | 4069,6 | 21,3 |
| 63 | 2752,4 | 14,0 | 77 | 2816,3 | 24,7 |
| 64 | 3515,8 | 15,3 | 78 | 3435,8 | 15,0 |
| 65 | 1082,5 | 17,7 | | | |

| **Nummer** | **Masse [Da]** | **CE-Zeit [min]** |
|---|---|---|
| 79 | 973,26 | 35,44 |
| 80 | 1128,54 | 25,66 |
| 81 | 1173,58 | 37,47 |
| 82 | 1184,6 | 26,43 |
| 83 | 1290,39 | 30,85 |
| 84 | 1338,66 | 23,96 |
| 85 | 1428,45 | 36,73 |
| 86 | 1450,6 | 37,47 |
| 87 | 1460,71 | 19,83 |
| 88 | 1498,46 | 35,31 |
| 89 | 1526,76 | 23,54 |
| 90 | 1588,77 | 30,24 |
| 91 | 1675,77 | 29,23 |
| 92 | 1703,91 | 33,67 |
| 93 | 1808,87 | 23,72 |
| 94 | 1863,96 | 44,01 |
| 95 | 1867,79 | 33,29 |
| 96 | 1925,9 | 23,2 |
| 97 | 2036,97 | 31,53 |
| 98 | 2114,05 | 31,59 |
| 99 | 2196,11 | 33,16 |
| 100 | 2212,06 | 33,36 |
| 101 | 2257,95 | 36,02 |
| 102 | 2544,06 | 26,14 |
| 103 | 2599,21 | 28,05 |
| 104 | 2761,38 | 21,47 |
| 105 | 3334,17 | 31,01 |
| 106 | 3361,41 | 24,32 |
| 107 | 3426,43 | 27,73 |
| 108 | 3765,51 | 20,18 |
| 109 | 3864,56 | 33,82 |
| 110 | 3870,8 | 33,47 |
| 111 | 4252,03 | 28,77 |
| 112 | 6211,93 | 20,27 |
| 113 | 6650,86 | 25,55 |
| 114 | 6820,37 | 21,03 |
| 115 | 16918,24 | 19,8 |

Mit der vorliegenden Erfindung ist es möglich, Prostatakrebs sehr frühzeitig zu diagnostizieren. Dadurch kann die Krankheit in einem frühen Stadium durch bekannte Verfahren therapiert werden. Die Erfindung ermöglicht weiterhin eine kostengünstige, schnelle und zuverlässige Diagnose bei zum Teil nicht oder nur minimal invasiven Eingriffen.

Ein weiterer Gegenstand der Erfindung ist die Differentialdiagnose zur Unterscheidung zwischen dem Prostatakarzinom und einer BPH. Die Differentialdiagnose kann durch die Verwendung der An- oder Abwesenheit von mindestens drei Polypeptidmarkern in einer Probe eines Individuums geschehen, wobei der Polypeptidmarker ausgewählt ist aus den Polypeptidmarkern 52-78, die durch die in Tabelle 2 angegebenen Molekularmassen und Migrationszeiten charakterisiert sind. Bevorzugt werden mehr Marker eingesetzt.

**Tabelle 2: Polypeptidmarker zur Differentialdiagnose von Prostatakrebs oder BPH, ihre Molekularmassen und Migrationszeiten.**

| **Nummer** | **Masse (Da)** | **CE-Zeit [min]** | **Nummer** | **Masse (Da)** | **CE-Zeit [min]** |
|---|---|---|---|---|---|
| 52 | 1636,8 | 27,2 | 66 | 1878,9 | 15,3 |
| 53 | 1412,6 | 22,1 | 67 | 3593,4 | 14,5 |
| 54 | 1579,7 | 26,4 | 68 | 1236,7 | 13,3 |
| 55 | 1390,5 | 35,0 | 69 | 2736,3 | 17,1 |
| 56 | 1196,6 | 15,8 | 70 | 2973,4 | 20,0 |
| 57 | 11041,4 | 16,6 | 71 | 1134,6 | 19,4 |
| 58 | 2971,4 | 17,9 | 72 | 2191,9 | 17,2 |
| 59 | 3136,6 | 20,0 | 73 | 2205,1 | 25,1 |
| 60 | 4409,9 | 14,2 | 74 | 3959,7 | 14,0 |
| 61 | 1494,7 | 26,4 | 75 | 1749,9 | 19,4 |
| 62 | 1833,9 | 24,2 | 76 | 4069,6 | 21,3 |
| 63 | 2752,4 | 14,0 | 77 | 2816,3 | 24,7 |
| 64 | 3515,8 | 15,3 | 78 | 3435,8 | 15,0 |
| 65 | 1082,5 | 17,7 | | | |

Die Migrationszeit wird mittels Kapillarelektrophorese (capillary electrophoresis, CE) - wie z.B. in Beispiel unter Punkt 2 ausgeführt- bestimmt. In diesem Beispiel wird eine 90 cm lange Glaskapillare mit einem inneren Durchmesser (ID) von 50 µm und einem äußeren Durchmesser (OD) von 360 µm bei einer angelegten Spannung von 30 kV betrieben. Als Laufmittel wird 30% Methanol, 0,5% Ameisensäure in Wasser verwendet.

Es ist bekannt, das die CE-Migrationszeit variieren kann. Dennoch ist die Reihenfolge, mit der die Polypeptidmarker eluieren, für jedes verwendete CE System unter den angegebenen Bedingungen typischerweise gleich. Um dennoch auftretende Unterschiede in der Migrationszeit auszugleichen, kann das System unter Verwendung von Standards, für die die Migrationszeiten genau bekannt sind, normiert werden. Diese Standards können z.B. die in den Beispielen angegebenen Polypeptide sein (siehe Beispiel Punkt 3).

Die Charakterisierung der Polypeptide, die in den Tabellen 1 bis 2 gezeigt sind, wurde mittels Kapillarelektrophorese-Massenspektrometrie (CE-MS) bestimmt, einem Verfahren, das z.B. ausführlich von Neuhoff et al. (Rapid communications in mass spectrometry, 2004, Bd. 20, Seite 149-156) beschrieben wurde. Die Variation der Molekülmassen zwischen einzelnen Messungen oder zwischen verschiedenen Massenspektrometern ist bei exakter Kalibrierung relativ klein, typischerweise im Bereich von ± 0,03%, vorzugsweise im Bereich von ± 0,01%.

Die erfindungsgemäßen Polypeptidmarker sind Proteine oder Peptide oder Abbauprodukte von Proteinen oder Peptiden. Sie können chemisch modifiziert sein, z.B. durch posttranslationale Modifikationen wie Glykolisierung, Phosphorylierung, Alkylierung oder Disulfidverbrückung, oder durch andere Reaktionen, z.B. im Rahmen des Abbaus, verändert sein. Darüber hinaus können die Polypeptidmarker auch im Rahmen der Aufreinigung der Proben chemisch verändert, z.B. oxidiert, sein.

Ausgehend von den Parametern, die die Polypeptidmarker bestimmen (Molekularmasse und Migrationszeit), ist es möglich, durch im Stand der Technik bekannte Verfahren die Sequenz der entsprechenden Polypeptide zu identifizieren.

Die erfindungsgemäßen Polypeptide (siehe Tabelle 1 bis 5) werden verwendet, um Prostatakrebs zu diagnostizieren. Unter Diagnose versteht man den Vorgang der Erkenntnisgewinnung durch die Zuordnung von Symptomen oder Phänomenen zu einer Krankheit oder Verletzung. Im vorliegenden Fall wird von der An- oder Abwesenheit bestimmter Polypeptidmarker auf das Vorliegen von Prostatakrebs geschlossen. Hierzu werden die erfindungsgemäßen Polypeptidmarker in einer Probe eines Individuums bestimmt, wobei, im Falle von Frequenzmarkern, ihre An- oder Abwesenheit auf das Vorliegen von Prostatakrebs schließen lässt. Die An- oder Abwesenheit eines Polypeptidmarkers kann durch jedes im Stand der Technik bekannte Verfahren gemessen werden. Verfahren, die verwendet werden können, sind weiter unten beispielhaft aufgeführt.

Ein Polypeptidmarker ist anwesend, wenn sein Messwert mindestens so hoch ist wie der Schwellenwert. Liegt sein Messwert darunter, ist der Polypeptidmarker abwesend. Der Schwellenwert kann entweder durch die Sensitivität des Messverfahrens (Nachweisgrenze) bestimmt werden oder anhand von Erfahrungen definiert werden.

Im Zusammenhang mit der vorliegenden Erfindung wird der Schwellenwert vorzugsweise überschritten, wenn der Messwert der Probe für eine bestimmte Molekularmasse mindestens doppelt so hoch ist, wie der einer Leerprobe (z.B. nur Puffer oder Lösungsmittel).

Der oder die Polypeptidmarker wird/werden in der Weise verwendet, dass seine/ihre An-oder Abwesenheit gemessen wird, wobei die An- oder Abwesenheit indikativ für Prostatakrebs ist (Frequenzmarker). So gibt es Polypeptidmarker, die typischerweise bei Patienten mit Prostatakrebs (krank) vorhanden sind, wie z.B. Polypeptidmarker Nr. 1 bis 11, jedoch bei Probanden ohne Prostatakrebs (Kontrolle) nicht vorhanden sind. Weiterhin gibt es Polypeptidmarker, die bei Individuen ohne Prostatakrebs vorhanden sind, jedoch bei Individuen mit Prostatakrebs seltener oder gar nicht auftreten, z.B. 12 bis 44 (Tabelle 3)

**Tabelle 3: Polypeptidmarker (Frequenzmarker) zur Diagnose von Prostatakrebs (PCA), ihre Molekularmassen und Migrationszeiten sowie ihre An- und Abwesenheit bei an Prostatakrebs erkrankten Patientengruppen sowie Kontrollgruppen als Faktor (1=100%, 0=0%; Probenaufarbeitung und Messung wie im Beispiel beschrieben).**

| **Nummer** | **Masse [Da]** | **CE-Zeit [min]** | **Differenz** | **Faktor PCA-Gruppe** | **Faktor Kontroll-Gruppe** |
|---|---|---|---|---|---|
| 1 | 1579,7 | 26,4 | D:0,75 | 1 | 0,25 |
| 2 | 1991,9 | 17,1 | D:0,66 | 0,88 | 0,22 |
| 3 | 1955,9 | 24,8 | D:0,65 | 1 | 0,35 |
| 4 | 1140,5 | 21,4 | D:0,57 | 1 | 0,43 |
| 5 | 1677,3 | 7,4 | D:0,57 | 0,63 | 0,06 |
| 6 | 10753,1 | 13,6 | D:0,54 | 0,88 | 0,33 |
| 7 | 1412,6 | 22,1 | D:0,53 | 0,75 | 0,22 |
| 8 | 1636,8 | 27,2 | D:0,53 | 0,63 | 0,1 |
| 9 | 1946,9 | 28,4 | D:0,53 | 0,75 | 0,22 |
| 10 | 5887,6 | 25,1 | D:0,51 | 1 | 0,49 |
| 11 | 12407,9 | 22,2 | D:0,51 | 0,75 | 0,24 |
| 12 | 1186,6 | 17,4 | D:-0,51 | 0,38 | 0,88 |
| 13 | 1240,6 | 23,3 | D:-0,51 | 0,25 | 0,76 |
| 14 | 1326,6 | 24,8 | D:-0,51 | 0,38 | 0,88 |
| 15 | 3802,1 | 30,1 | D:-0,51 | 0,38 | 0,88 |
| 16 | 1749,9 | 19,4 | D:-0,52 | 0,13 | 0,65 |
| 17 | 2216,1 | 31,0 | D:-0,52 | 0,13 | 0,65 |
| 18 | 1069,5 | 22,7 | D:-0,53 | 0 | 0,53 |
| 19 | 1341,6 | 26,6 | D:-0,53 | 0 | 0,53 |
| 20 | 1353,7 | 21,8 | D:-0,53 | 0,25 | 0,78 |
| 21 | 1716,8 | 24,6 | D:-0,53 | 0,25 | 0,78 |
| 22 | 2939,1 | 31,1 | D:-0,53 | 0,38 | 0,9 |
| 23 | 3002,0 | 19,2 | D:-0,53 | 0,25 | 0,78 |
| 24 | 1110,4 | 31,5 | D:-0,54 | 0,13 | 0,67 |
| 25 | 1496,7 | 26,5 | D:-0,54 | 0,13 | 0,67 |
| 26 | 1825,8 | 28,4 | D:-0,54 | 0,13 | 0,67 |
| 27 | 1180,5 | 33,9 | D:-0,55 | 0 | 0,55 |
| 28 | 2421,1 | 32,5 | D:-0,55 | 0 | 0,55 |
| 29 | 4345,9 | 30,6 | D:-0,55 | 0 | 0,55 |
| 30 | 2077,1 | 16,9 | D:-0,56 | 0,13 | 0,69 |
| 31 | 1134,6 | 19,4 | D:-0,57 | 0,25 | 0,82 |
| 32 | 1444,8 | 13,5 | D:-0,58 | 0,13 | 0,71 |
| 33 | 1046,5 | 21,9 | D:-0,59 | 0 | 0,59 |
| 34 | 1992,2 | 16,8 | D:-0,59 | 0 | 0,59 |
| 35 | 4069,6 | 21,3 | D:-0,59 | 0 | 0,59 |
| 36 | 1191,5 | 34,5 | D:-0,61 | 0 | 0,61 |
| 37 | 1239,5 | 32,5 | D:-0,61 | 0 | 0,61 |
| 38 | 2191,9 | 17,2 | D:-0,61 | 0,25 | 0,86 |
| 39 | 1865,8 | 30,0 | D:-0,62 | 0,13 | 0,75 |
| 40 | 1265,6 | 23,4 | D:-0,65 | 0,25 | 0,9 |
| 41 | 1936,1 | 10,5 | D:-0,65 | 0 | 0,65 |
| 42 | 911,3 | 31,9 | D:-0,69 | 0 | 0,69 |
| 43 | 1494,7 | 26,4 | D:-0,70 | 0,13 | 0,82 |
| 44 | 1209,6 | 22,5 | D:-0,71 | 0 | 0,71 |

Zusätzlich oder auch alternativ zu den Frequenzmarkern (Bestimmung der An- oder Abwesenheit) können auch die in Tabelle 4 angegebenen Amplitudenmarker zur Diagnose von Prostatakrebs verwendet werden (Nummer 45-51, 79-115). Amplitudenmarker werden in der Weise verwendet, das nicht die An oder Abwesenheit entscheidend ist, sondern die Höhe des Signals (die Amplitude) bei Anwesenheit des Signals in beiden Gruppen entscheidet. In Tabelle 5 sind die mittleren Amplituden der entsprechenden Signale (charakterisiert über Masse and Migrationszeit) über alle gemessenen Proben angegeben. Um eine Vergleichbarkeit zwischen unterschiedlich konzentrierten Proben oder unterschiedlichen Messmethoden zu erreichen werden alle Peptidsignale einer Probe auf eine Gesamtamplitude von 1 Million Counts normiert. Die jeweiligen mittleren Amplituden der Einzelmarker sind daher als parts per million (ppm) angegeben. Alle verwendeten Gruppen bestehen aus mindestens 20 einzelnen Patienten- oder Kontrollproben, um eine verlässliche mittlere Amplitude zu erhalten. Die Entscheidung zu einer Diagnose (PCA oder nicht) fällt dabei je nachdem, wie hoch die Amplitude der jeweiligen Polypeptidmarker in der Patientenprobe im Vergleich zu den mittleren Amplituden in der Kontrollgruppe bzw. der PCA-Gruppe ist. Entspricht die Amplitude eher den mittleren Amplituden der PCA-Gruppe, ist von einem PCA auszugehen, entspricht sie eher den mittleren Amplituden der Kontroll-Gruppe, ist nicht von einem PCA auszugehen. Eine genauere Definition soll anhand von Marker Nr. 48 (Tabelle 4) gegeben werden. Die mittlere Amplitude des Markers ist bei PCA deutlich erhöht (2600 ppm gegen 1600 ppm in der Kontrollgruppe). Liegt nun in einer Patientenprobe der Wert für diesen Marker bei 0 bis 1600 ppm, bzw. maximal 20% darüber, also 0 bis 1920 ppm, gehört diese Probe zur Kontrollgruppe. Liegt der Wert bei 2600 ppm, bzw. 20% darunter, oder höher, also zwischen 2080 und sehr hohen Werten, ist von PCA auszugehen.

Je geringer der Abstand zwischen den Amplituden der Kontrollgruppe und der Alzheimer Gruppe, desto dichter muss der Wert, der zwischen den beiden Referenzwerten liegt, an einem Referenzwert liegen.

Eine Möglichkeit ist, den Bereich zwischen den mittleren Amplituden in drei Teile zu zerlegen. Liegt der Wert im unteren Drittel, ist dies indikativ für den unteren Wert, liegt der Wert im oberen Drittel, ist dies indikativ für den oberen Wert. Liegt er im mittleren Drittel, ist keine Aussage hinsichtlich dieses Markers möglich.

**Tabelle 4: Amplitudenmarker**

| **Nummer** | **Masse [Da]** | **CE-Zeit [min]** | **Mittlere Amplitude in PCA-Proben [ppm]** | **Mittlere Amplitude in Kontroll-Proben [ppm]** |
|---|---|---|---|---|
| 45 | 1193,3 | 34,2 | 24000 | 11000 |
| 46 | 1235,4 | 34,3 | 160 | 370 |
| 47 | 1390,5 | 35,0 | 22000 | 12000 |
| 48 | 2292,1 | 23,7 | 2600 | 1600 |
| 49 | 2736,3 | 17,1 | 120 | 350 |
| 50 | 3385,5 | 21,0 | 2600 | 1700 |
| 51 | 3945,2 | 21,4 | 260 | 120 |
| | | | | |

| **Nummer** | **Masse [Da]** | **CE-Zeit** | **Mittl. Amplitude PCa-Proben ppm** | **Mittl. Amplitude in Kontroll-Proben ppm** |
|---|---|---|---|---|
| 79 | 973,26 | 35,44 | 112,27 | 94,56 |
| 80 | 1128,54 | 25,66 | 104,1 | 135,92 |
| 81 | 1173,58 | 37,47 | 163,08 | 174,37 |
| 82 | 1184,6 | 26,43 | 61,33 | 53,82 |
| 83 | 1290,39 | 30,85 | 425,14 | 360,22 |
| 84 | 1338,66 | 23,96 | 116,85 | 223,77 |
| 85 | 1428,45 | 36,73 | 2325,09 | 1972,91 |
| 86 | 1450,6 | 37,47 | 348,86 | 350,46 |
| 87 | 1460,71 | 19,83 | 204,2 | 216,45 |
| 88 | 1498,46 | 35,31 | 115,26 | 94,66 |
| 89 | 1526,76 | 23,54 | 94,2 | 155,52 |
| 90 | 1588,77 | 30,24 | 504,83 | 565,2 |
| 91 | 1675,77 | 29,23 | 104,37 | 83,95 |
| 92 | 1703,91 | 33,67 | 326,26 | 495,27 |
| 93 | 1808,87 | 23,72 | 189,86 | 198,11 |
| 94 | 1863,96 | 44,01 | 989,35 | 1411,72 |
| 95 | 1867,79 | 33,29 | 117,97 | 108,75 |
| 96 | 1925,9 | 23,2 | 148,34 | 145,13 |
| 97 | 2036,97 | 31,53 | 74,45 | 77,29 |
| 98 | 2114,05 | 31,59 | 135,48 | 121,91 |
| 99 | 2196,11 | 33,16 | 479,36 | 390,89 |
| 100 | 2212,06 | 33,36 | 336,96 | 397,62 |
| 101 | 2257,95 | 36,02 | 365,38 | 567,62 |
| 102 | 2544,06 | 26,14 | 77,74 | 121,23 |
| 103 | 2599,21 | 28,05 | 797,34 | 915,08 |
| 104 | 2761,38 | 21,47 | 744,83 | 616,6 |
| 105 | 3334,17 | 31,01 | 105,87 | 127,64 |
| 106 | 3361,41 | 24,32 | 143,28 | 108,31 |
| 107 | 3426,43 | 27,73 | 149,46 | 116,27 |
| 108 | 3765,51 | 20,18 | 375,75 | 309,53 |
| 109 | 3864,56 | 33,82 | 271,44 | 162,02 |
| 110 | 3870,8 | 33,47 | 71,63 | 78,31 |
| 111 | 4252,03 | 28,77 | 388,07 | 447,93 |
| 112 | 6211,93 | 20,27 | 492,21 | 400,75 |
| 113 | 6650,86 | 25,55 | 233,38 | 232,51 |
| 114 | 6820,37 | 21,03 | 258,88 | 227,62 |
| 115 | 16918,24 | 19,8 | 605,64 | 324,52 |

Zur Differentialdiagnose zwischen PCA und BPH zeigt Tabelle 5 Polypeptidmarker, die typischerweise bei Patienten mit Prostatakarzinom vorhanden sind, wie z.B. die Marker Nr. 52 bis 58, jedoch bei Probanden mit BPH nicht oder nur selten vorhanden sind. Weiterhin gibt es Polypeptidmarker, die bei Individuen mit BPH vorhanden sind, jedoch bei Individuen mit PCA seltener oder gar nicht auftreten, z.B. die Polypeptidmarker Nr. 59 bis 78.

**Tabelle 5: Polypeptidmarker (Frequenzmarker) zur Differentialdiagnose zwischen Prostatakrebs (PCA) und BPH, ihre Molekularmassen und Migrationszeiten sowie ihre An- und Abwesenheit bei an Prostatakrebs erkrankten Patientengruppen sowie der BPH-Gruppe als Faktor (1=100%, 0=0%; Probenaufarbeitung und Messung wie im Beispiel beschrieben).**

| **Nummer** | **Massen (Da)** | **CE-Zeit [min]** | **Differenz** | **Faktor PCA-Gruppe** | **Faktor BP-Gruppe** |
|---|---|---|---|---|---|
| 52 | 1636,8 | 27,2 | D:0,63 | 0,63 | 0 |
| 53 | 1412,6 | 22,1 | D:0,57 | 0,75 | 0,18 |
| 54 | 1579,7 | 26,4 | D:0,55 | 1 | 0,45 |
| 55 | 1390,5 | 35,0 | D:0,53 | 0,63 | 0,09 |
| 56 | 1196,6 | 15,8 | D:0,51 | 0,88 | 0,36 |
| 57 | 11041,4 | 16,6 | D:0,51 | 0,88 | 0,36 |
| 58 | 2971,4 | 17,9 | D:0,50 | 0,5 | 0 |
| 59 | 3136,6 | 20,0 | D:-0,50 | 0,5 | 1 |
| 60 | 4409,9 | 14,2 | D:-0,50 | 0,5 | 1 |
| 61 | 1494,7 | 26,4 | D:-0,51 | 0,13 | 0,64 |
| 62 | 1833,9 | 24,2 | D:-0,51 | 0,13 | 0,64 |
| 63 | 2752,4 | 14,0 | D:-0,51 | 0,13 | 0,64 |
| 64 | 3515,8 | 15,3 | D:-0,51 | 0,13 | 0,64 |
| 65 | 1082,5 | 17,7 | D:-0,53 | 0,38 | 0,91 |
| 66 | 1878,9 | 15,3 | D:-0,53 | 0,38 | 0,91 |
| 67 | 3593,4 | 14,5 | D:-0,53 | 0,38 | 0,91 |
| 68 | 1236,7 | 13,3 | D:-0,55 | 0 | 0,55 |
| 69 | 2736,3 | 17,1 | D:-0,55 | 0 | 0,55 |
| 70 | 2973,4 | 20,0 | D:-0,55 | 0 | 0,55 |
| 71 | 1134,6 | 19,4 | D:-0,57 | 0,25 | 0,82 |
| 72 | 2191,9 | 17,2 | D:-0,57 | 0,25 | 0,82 |
| 73 | 2205,1 | 25,1 | D:-0,57 | 0,25 | 0,82 |
| 74 | 3959,7 | 14,0 | D:-0,57 | 0,25 | 0,82 |
| 75 | 1749,9 | 19,4 | D:-0,60 | 0,13 | 0,73 |
| 76 | 4069,6 | 21,3 | D:-0,64 | 0 | 0,64 |
| 77 | 2816,3 | 24,7 | D:-0,73 | 0 | 0,73 |
| 78 | 3435,8 | 15,0 | D:-0,82 | 0 | 0,82 |

Das Individuum, von dem die Probe stammt, in der die An- oder Abwesenheit oder die Amplitude eines oder mehrerer Polypeptid marker bestimmt wird, kann jedes Individuum sein, das an Prostatakrebs leiden kann, z.B. ein Tier oder ein Mensch. Vorzugsweise handelt es sich bei dem Individuum um ein Säugetier, wie z.B. einen Hund oder ein Pferd, am meisten bevorzugt handelt es sich um einen Menschen.

Für die Anwendung der Erfindung wird nicht nur ein Polypeptidmarker, sondern eine Kombination von Markern verwendet, um Prostatakrebs zu diagnostizieren. Dabei wird durch ihre An- oder Abwesenheit und/oder die Höhe der Amplitude auf das Vorliegen von Prostatakrebs geschlossen. Durch Vergleich einer Mehrzahl von Polypeptidmarkern kann die Verfälschung des Gesamtergebnisses durch einzelne individuelle Abweichungen von der typischen Anwesenheitswahrscheinlichkeit im Kranken oder Kontrollindividuum reduziert oder vermieden werden.

Bei der Probe, in der die An- oder Abwesenheit oder die Amplitude des oder der erfindungsgemäßen Polypeptidmarker gemessen werden, kann es sich um jede Probe handeln, die aus dem Körper des Individuums gewonnen wird. Bei der Probe handelt es sich um eine Probe, die über eine Polypeptidzusammensetzung verfügt, die geeignet ist, Aussagen über den Zustand des Individuums (Prostatakrebs oder nicht) zu treffen. Beispielsweise kann es sich um Urin, Sperma, Samenflüssigkeit (Sperma ohne Spermien) handeln. Vorzugsweise handelt es sich um eine Flüssigprobe.

In einer bevorzugten Ausführungsform handelt es sich bei der Probe um eine Urinprobe.

Urinproben können wie im Stand der Technik bekannt genommen werden. Vorzugsweise wird im Zusammenhang mit der vorliegenden Erfindung eine Mittelstrahlurinprobe verwendet. Die Urinprobe kann z.B. mittels eines Katheters oder auch mit Hilfe eines Urinierungsapparates, wie in WO 01/74275 beschrieben, entnommen werden.

Die An- oder Abwesenheit eines Polypeptid markers in der Probe kann durch jedes im Stand der Technik bekannte Verfahren, das zur Messung von Polypeptid markern geeignet ist, bestimmt werden. Dem Fachmann sind solche Verfahren bekannt. Grundsätzlich kann die An- oder Abwesenheit eines Polypeptid markers durch direkte Verfahren, wie z.B. Massenspektrometrie, oder indirekte Verfahren, wie z.B. mittels Liganden, bestimmt werden.

Falls erforderlich oder wünschenswert kann die Probe des Individuums, z.B. die Urinprobe, vor der Messung der An- oder Abwesenheit des oder der Polypeptidmarker durch jedes geeignete Mittel vorbehandelt und z.B. aufgereinigt oder aufgetrennt werden. Die Behandlung kann z.B. eine Aufreinigung, Trennung, Verdünnung oder Konzentrierung umfassen. Die Verfahren können beispielsweise eine Zentrifugation, Filtration, Ultrafiltration, Dialyse, eine Fällung oder chromatographische Verfahren wie Affinitätstrennung oder Trennung mittels Ionenaustauscher-chromatographie, oder eine elektrophoretische Trennung sein. Besondere Beispiele hierfür sind Gelelektrophorese, zweidimensionale Polyacrylamidgelelektrophorese (2D-PAGE), Kapillarelektrophorese, Metallaffinitätschromatographie, immobilisierte Metallaffinitätschromatographie (IMAC), Affinitätschromatographie auf der Basis von Lektinen, Flüssigchromatographie, Hochleistungsflüssigchromatographie (HPLC), Normal- und Umkehrphasen-HPLC, Kationenaustauscherchromatographie und selektive Bindung an Oberflächen. Alle diese Verfahren sind dem Fachmann gut bekannt und der Fachmann wird das Verfahren in Abhängigkeit von der verwendeten Probe und dem Verfahren zur Bestimmung der An-oder Abwesenheit des oder der Polypeptidmarker auswählen können.

In einer Ausführungsform der Erfindung wird die Probe vor ihrer Messung mittels Kapillarelektrophorese aufgetrennt, mittels Ultrazentrifugation gereinigt und/oder mittels Ultrafiltration in Fraktionen, die Polypeptidmarker bestimmter molekularer Größe enthalten, aufgetrennt.

Vorzugsweise wird ein massenspektrometrisches Verfahren verwendet, um die An- oder Abwesenheit eines Polypeptid markers zu bestimmen, wobei diesem Verfahren eine Aufreinigung oder Auftrennung der Probe vorgeschaltet werden kann. Die massenspektrometrische Analyse besitzt gegenüber den derzeit gängigen Verfahren den Vorteil, dass die Konzentration vieler (>100) Polypeptide einer Probe mittels einer einzigen Analyse bestimmt werden kann. Jeder Typ eines Massenspektrometers kann verwendet werden. Mit der Massenspektrometrie ist es möglich, routinemäßig 10 fmol eines Polypeptidmarkers, also 0,1 ng eines 10 kDa Proteins mit einer Messgenauigkeit von ca. ±0,01% aus einem komplexen Gemisch zu vermessen. Bei Massenspektrometern ist eine Ionen-bildende Einheit mit einem geeigneten Analysegerät gekoppelt. Zum Beispiel werden meistens Elektrospray-Ionisations (ESI) Interfaces verwendet, um Ionen aus Flüssigproben zu vermessen, wohingegen die Matrix-assisted-laser-desorption/ionisation (MALDI) Technik verwendet wird, um Ionen aus mit einer Matrix kristallisierten Probe zu vermessen. Zur Analyse der entstandenen Ionen können z.B. Quadrupole, Ionenfallen oder Time-of-flight (TOF) Analysatoren verwendet werden.

Bei der Elektrosprayionisation (ESI) werden die in Lösung vorliegenden Moleküle u.a. unter dem Einfluss von Hochspannung (z.B. 1-8 kV) versprüht, wobei sich geladenen Tröpfchen bilden, die durch Verdampfen des Lösungsmittels kleiner werden. Schließlich kommt es durch sog. Coulomb-Explosionen zur Bildung freier Ionen, die dann analysiert und detektiert werden können.

Bei der Analyse der Ionen mittels TOF wird eine bestimmte Beschleunigungsspannung angelegt, die den Ionen eine gleich große kinetische Energie verleiht. Dann wird sehr genau die Zeit gemessen, die die jeweiligen Ionen benötigen, um eine Driftstrecke durch das Flugrohr zurückzulegen. Da bei gleicher kinetische Energie die Geschwindigkeit der Ionen von Ihrer Masse abhängt, kann diese somit bestimmt werden. TOF-Analysatoren haben eine sehr hohe Scan-Geschwindigkeit und erreichen eine sehr hohe Auflösung.

Bevorzugte Verfahren zur Bestimmung der An- oder Abwesenheit von Polypeptidmarkern schließen Gasphasenionenspektrometrie, wie Laserdesorptions /Ionisations-Massenspektrometrie, MALDI-TOF-MS, SELDI-TOF-MS (Surface enhanced laser desorption ionisation), LC-MS (Liquid chromatography- mass spectrometry), 2D-PAGE-MS und Kapillarelektrophorese-Massenspektrometrie (CE-MS) ein. Alle genannten Verfahren sind dem Fachmann bekannt.

Ein besonders bevorzugtes Verfahren ist CE-MS, in welchem die Kapillarelektrophorese mit Massenspektrometrie gekoppelt wird. Dieses Verfahren ist ausführlich z.B. in der deutschen Patentanmeldung DE 10021737, bei Kaiser et al. *(*J. Chromatogr. A, 2003, Bd. 1013:157-171, sowie Electrophoresis, 2004, 25:2044-2055) und bei Wittke et al. (J. Chromatogr. A, 2003, 1013:173-181) beschrieben. Die CE-MS Technik erlaubt, das Vorhandensein einiger Hunderter Polypeptid marker einer Probe gleichzeitig in kurzer Zeit, einem geringen Volumen und hoher Sensitivität zu bestimmen. Nachdem eine Probe vermessen wurde, wird ein Muster der gemessenen Polypeptid marker hergestellt. Dieses kann mit Referenzmustern von kranken bzw. gesunden Individuen verglichen werden. In den meisten Fällen ist es ausreichend, eine begrenzte Anzahl von Polypeptidmarkern für die Diagnose von Prostatakrebs und die Differentialdiagnose zwischen Prostatakrebs und BPH zu verwenden. Weiter bevorzugt ist ein CE-MS Verfahren, das CE online an ein ESI-TOF-MS gekoppelt, einschließt.

Für CE-MS ist die Verwendung von flüchtigen Lösungsmitteln bevorzugt, außerdem arbeitet man am besten unter im Wesentlichen salzfreien Bedingungen. Beispiele geeigneter Lösungsmittel umfassen Acetonitril, Methanol und ähnliche. Die Lösungsmittel können mit Wasser verdünnt und mit einer schwachen Säure (z.B. 0,1% bis 1% Ameisensäure) versetzt sein, um den Analyten, vorzugsweise die Polypeptide, zu protonieren.

Mit der Kapillarelektrophorese ist es möglich, Moleküle nach ihrer Ladung und Größe zu trennen. Neutrale Teilchen wandern beim Anlegen eines Stromes mit der Geschwindigkeit des elektroosmotischen Flusses, Kationen werden zur Kathode beschleunigt und Anionen verzögert. Der Vorteil von Kapillaren in der Elektrophorese besteht im günstigen Verhältnis von Oberfläche zu Volumen, was einen guten Abtransport der beim Stromfluss entstehenden Jouleschen Wärme ermöglicht. Dies wiederum erlaubt das Anlegen hoher Spannungen (üblicherweise bis 30 kV) und damit eine hohe Trennleistung und kurze Analysezeiten.

Bei der Kapillarelektrophorese werden normalerweise Quarzglaskapillaren mit Innendurchmessern von typischerweise 50 bis 75 µm eingesetzt. Die verwendeten Längen betragen 30-100 cm. Darüber hinaus bestehen die Kapillaren in der Regel aus kunststoffumhüllten Quarzglas. Die Kapillaren können sowohl unbehandelt sei, d.h. auf der Innenseite ihre hydrophilen Gruppen zeigen, als auch auf der Innenseite beschichtet sein. Eine hydrophobe Beschichtung kann verwendet werden, um die Auflösung zu verbessern. Zusätzlich zur Spannung kann auch ein Druck angelegt werden, der typischerweise im Bereich von 0-1 psi liegt. Der Druck kann dabei auch erst während der Trennung angelegt oder währenddessen verändert werden.

In einem bevorzugten Verfahren zur Messung von Polypeptidmarkern werden die Marker der Probe mittels Kapillarelektrophorese getrennt, anschließend direkt ionisiert und online in ein daran gekoppeltes Massenspektrometer zur Detektion überführt.

In dem erfindungsgemäßen Verfahren können vorteilhafter Weise mehrere Polypeptidmarker zur Diagnose von Prostatakrebs verwendet werden. Insbesondere können mindestens drei Polypeptidmarker verwendet werden, beispielsweise die Marker 1, 2 und 3; 1, 2 und 4; usw.

Mehr bevorzugt ist die Verwendung von mindestens 4, 5, oder 6 Markern.

Noch mehr bevorzugt ist die Verwendung von mindestens 11 Markern, beispielsweise die Marker 1 bis 11.

Am meisten bevorzugt ist die Verwendung aller in den Tabellen 1 bzw. 3 aufgeführten Marker.

Auch zur Differentialdiagnose zwischen PCA und BPH können mehrere Marker verwendet werden. Insbesondere können mindestens drei Polypeptidmarker verwendet werden, beispielsweise die Marker 45, 46 und 47; 45, 46 und 48; usw.

Mehr bevorzugt ist die Verwendung von mindestens 4, 5, oder 6 Markern.

Noch mehr bevorzugt ist die Verwendung von mindestens 7 Markern, beispielsweise die Marker 1 bis 7.

Am meisten bevorzugt ist die Verwendung aller 27 in den Tabellen 2 bzw. 4 aufgeführten Marker.

Um die Wahrscheinlichkeit für das Vorliegen von Prostatakrebs bei Verwendung mehrerer Marker zu bestimmen, können dem Fachmann bekannte statistische Verfahren verwendet werden. Beispielsweise kann das von Weissinger et al. (Kidney Int., 2004, 65:2426-2434) beschriebene Random-Forests-Verfahren unter Verwendung eines Computerprogramms wie z.B. S-Plus verwendet werden.

Beispiel:

### 1. Probenvorbereitung:

Zur Detektion der Polypeptidmarker für Prostatakrebs wurde Urin verwendet. Urin wurde von gesunden Spendern (Vergleichsgruppe) sowie Patienten, die an Prostatakrebs oder BPH leiden, abgenommen.

Für die nachfolgende CE-MS Messung mussten die auch in Urin von Patienten in höherer Konzentration vorkommenden Proteine wie Albumin und Immunoglobuline durch Ultrafiltration abgetrennt werden. Dazu wurden 500 µl Urin entnommen und mit 2 ml Filtrationspuffer (4M Harnstoff, 0,1M NaCl, 0,01% Ammoniak) versetzt. Diese 2,5 ml Probenvolumen wurden ultrafiltriert (Amicon 30 kDa, Millipore, Bedford USA). Die UF wurde bei 3000 U/min in einer Zentrifuge durchgeführt bis 2 ml Ultrafiltrat erhalten wurden.

Die erhaltenen 2 ml Filtrat wurden dann auf eine Pharmacia C-2 Säule aufgetragen (Pharmacia, Uppsala, Schweden), um Harnstoff, Salze und andere störende Komponenten zu entfernen. Die gebundenen Polypeptide wurden dann mit 50% Acetonitril, 0,5% Ameisensäure in Wasser von der C-2 Säule eluiert und lyophillisiert. Zur CE-MS Messung wurden die Polypeptide dann mit 20 µl Wasser (HPLC-Reinheit, Merck) resuspendiert.

### 2. CE-MS Messung:

Die CE-MS Messungen wurden mit einem Kapillarelektrophoresesystem von Beckman Coulter (P/ACE MDQ System; Beckman Coulter Inc, Fullerton, USA) und einem ESI-TOF Massenspektrometer von Bruker (micro-TOF MS, Bruker Daltonik, Bremen, D) durchgeführt.

Die CE Kapillaren wurden von Beckman Coulter bezogen, sie hatten einen ID/OD von 50/360 µm und eine Länge von 90 cm. Die mobile Phase für die CE Trennung bestand aus 30% Methanol und 0,5% Ameisensäure in Wasser. Für den "Sheath-Flow" am MS wurde 30% Isopropanol mit 0,5% Ameisensäure verwendet, hier mit einer Flussrate von 2 µl/min. Die Kopplung von CE und MS wurde durch ein CE-ESI-MS Sprayer Kit (Agilent Technologies, Waldbronn, DE) realisiert.

Um die Probe zu injizieren, wurde 1 bis max. 6 psi Druck angelegt, die Dauer der Injektion betrug 99 Sekunden. Mit diesen Parametern wurden ca. 150 nl der Probe in die Kapillare injiziert, dieses entspricht ca. 10% des Kapillarvolumens. Um die Probe in der Kapillare aufzukonzentrieren wurde eine "Stacking"-Technik verwendet. Dabei wird vor der Probeninjektion für 7 Sek. (bei 1 psi) eine 1M NH₃ Lösung injiziert, nach der Probeninjektion für 5 Sek. eine 2M Ameisensäurelösung. Nach Anlegen der Trennspannung (30 kV) werden die Analyten zwischen diesen Lösungen automatisch aufkonzentriert.

Die folgende CE-Trennung wurde mit einer Druckmethode durchgeführt: 40 Minuten mit 0 psi, dann für 2 min 0,1 psi, für 2 min 0,2 psi, für 2 min 0,3 psi, für 2 min 0,4 psi, abschließend 32 min bei 0,5 psi. Die Gesamtdauer eines Trennlaufes betrug damit 80 Minuten.

Um auf der Seite des MS eine möglichst gute Signalintensität zu erhalten, wurde das "Nebulizer Gas" auf den niedrigsten möglichen Wert eingestellt. Die an der Spraynadel angelegte Spannung zur Erzeugung des Elektrosprays betrug 3700 - 4100 V. Die übrigen Einstellungen am Massenspektrometer wurden gemäß Anweisung des Herstellers für Peptiddetektion optimiert. Die Spektren wurden über einen Massenbereich von m/z 400 bis m/z 3000 aufgenommen und alle 3 Sek. akkumuliert.

### 3. Standards für die CE-Messunq

Zur Kontrolle und Kalibrierung der CE-Messung wurden die folgenden Proteine bzw. Polypeptide eingesetzt, welche unter den gewählten Bedingungen durch die unten aufgeführten CE-Migrationszeiten charakterisiert sind:

| Protein/Polypeptid | Migrationszeit |
|---|---|
| Aprotinin, (SIGMA, Taufkirchen, DE; Kat.Nr. A1153) | 9,2 min |
| Ribonuclease, SIGMA, Taufkirchen, DE; Kat.Nr.; R4875 | 10,9 min |
| Lysozym, SIGMA, Taufkirchen, DE; Kat.Nr.; L7651 | 8,9 min |
| "REV", Sequenz: REVQSKIGYGRQIIS | 15,6 min |
| "ELM", Sequenz: ELMTGELPYSHINNRDQIIFMVGR | 23,4 min |
| "KINCON", Sequenz: TGSLPYSHIGSRDQIIFMVGR | 20,0 min |
| "GIVLY" Sequenz: GIVLYELMTGELPYSHIN | 36,8 min |

Die Proteine/Polypeptide werden jeweils in einer Konzentration von 10 pmol/µl in Wasser eingesetzt. "REV", "ELM", "KINCON" und "GIVLY" stellen synthetische Peptide dar.

Die Molekularmassen der Peptide sowie die in der MS sichtbaren m/z Verhältnisse der einzelnen Ladungszustände sind in der folgenden Tabelle angegeben:

| **H (mono)** | 1,0079 | 1,0079 | 1,0079 | 1,0079 | 1,0079 | 1,0079 | 1,0079 |
|---|---|---|---|---|---|---|---|
| m/z | **Aprotinin** | **Ribonuclease** | **Lysozym** | **REV** | **KINCON** | **ELM** | **GIVLY** |
| | Mono Mass | Mono Mass | Mono Mass | Mono Mass | Mono Mass | Mono Mass | Mono Mass |
| 0 | 6513,09 | 13681,32 | 14303,88 | 1732,96 | 2333,19 | 2832,41 | 2048,03 |
| 1 | 6514,0979 | 13682,328 | 14304,888 | 1733,9679 | 2334,1979 | 2833,4179 | 2049,0379 |
| 2 | 3257,5529 | 6841,6679 | 7152,9479 | 867,4879 | 1167,6029 | 1417,2129 | 1025,0229 |
| 3 | 2172,0379 | 4561,4479 | 4768,9679 | 578,6612 | 778,7379 | 945,1446 | 683,6846 |
| 4 | 1629,2804 | 3421,3379 | 3576,9779 | 434,2479 | 584,3054 | 709,1104 | 513,0154 |
| 5 | 1303,6259 | 2737,2719 | 2861,7839 | 347,5999 | 467,6459 | 567,4899 | 410,6139 |
| 6 | 1086,5229 | 2281,2279 | 2384,9879 | 289,8346 | 389,8729 | 473,0762 | 342,3462 |
| 7 | 931,4494 | 1955,4822 | 2044,4193 | 248,5736 | 334,3208 | 405,6379 | 293,5836 |
| 8 | 815,1442 | 1711,1729 | 1788,9929 | 217,6279 | 292,6567 | 355,0592 | 257,0117 |
| 9 | 724,6846 | 1521,1546 | 1590,3279 | 193,559 | 260,2512 | 315,7201 | 228,5668 |
| 10 | 652,3169 | 1369,1399 | 1431,3959 | 174,3039 | 234,3269 | 284,2489 | 205,8109 |
| 11 | 593,107 | 1244,7643 | 1301,3606 | 158,5497 | 213,1161 | 258,4997 | 187,1924 |
| 12 | 543,7654 | 1141,1179 | 1192,9979 | 145,4212 | 195,4404 | 237,0421 | 171,6771 |
| 13 | 502,0148 | 1053,4171 | 1101,3063 | 134,3125 | 180,4841 | 218,8856 | 158,5486 |

Beispiel 4. Leistungsfähigkeit der in WO 01/25791 beschriebenen Marker.

In der WO 01/25791 sind elf Marker erwähnt (siehe dort Anspruch 2), die Genauigkeit der Massenbestimmung wird mit 0,5% angegeben. Geht man davon aus, dass es sich nur um Fragmente von Semenogelin I handelt, ergeben sich folgende Anzahlen an möglichen Fragmenten aus Semenogelin:

| **Molekularmassen der Biomarkerpolypeptide für Prostatakarzinom [g/mol]** | **Zahl möglicher Fragmente aus Seminogelin I** | |
|---|---|---|
| | **Massenabweichung 0,5 %** | **Massenabweichung 0,03%** |
| 2776 | 100 | 8 |
| 4423 | 182 | 16 |
| 4480 | 173 | 8 |
| 5753 | 212 | 11 |
| 6098 | 214 | 7 |
| 6270 | 217 | 19 |
| 6998 | 248 | 12 |
| 7843 | 270 | 11 |
| 8030 | 265 | 23 |
| 8240 | 281 | 12 |
| 8714 | 299 | 23 |

Weiterhin offenbart WO 01/25791 Marker, die gegen das Vorliegen eines Prostatakarzinoms sprechend. Die Anzahl möglicher Fragmente ist in der folgenden Tabelle enthalten:

| **Molekularmassen der Biomarkerpolypeptide gegen Prostatakerpolypeptide [g/mol]** | **Zahl möglicher Fragmente aus Seminogelin I** | |
|---|---|---|
| | **Massenabweichung 0,5%** | **Massenabweichung 0,03%** |
| 2095 | 83 | 2 |
| 2276 | 84 | 5 |
| 2530 | 99 | 6 |
| 3030 | 106 | 4 |
| 3038 | 116 | 10 |
| 3224 | 127 | 7 |
| 3600 | 139 | 6 |
| 3835 | 146 | 9 |
| 3915 | 150 | 16 |
| 3933 | 153 | 23 |
| 4175 | 161 | 14 |

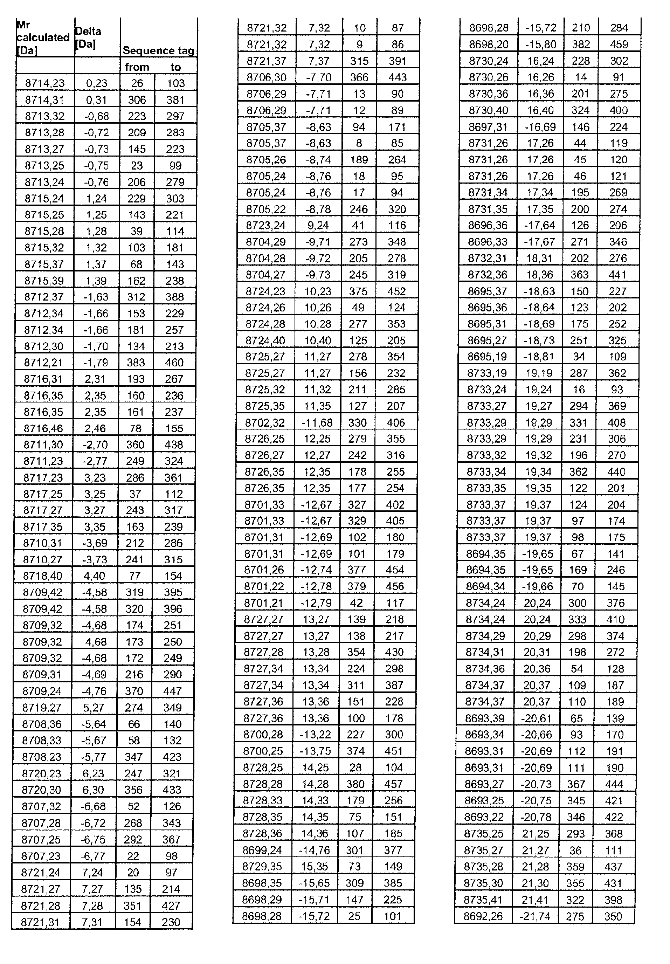

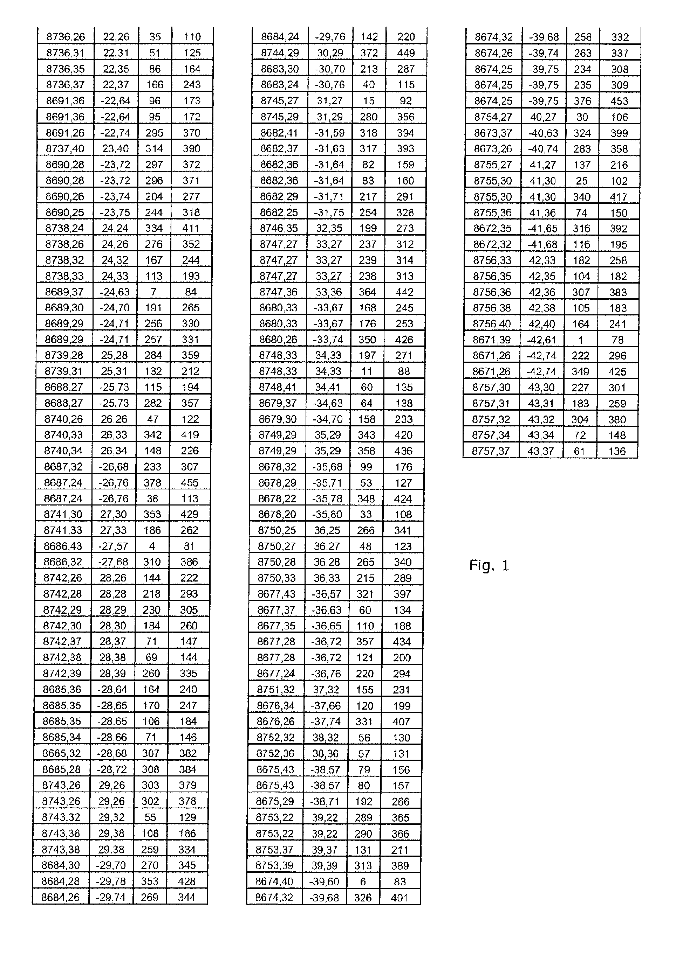

Figur 1 zeigt beispielhaft für eine Masse die Vielzahl an Sequenzen, die in diesem Gewichtsbereich möglich sind.

Im nächsten Schritt wurde versucht, entsprechende Proteine in Urinproben von Patienten mit Prostatakarzinom oder BPH zu analysieren. Dabei konnten nur sechs Marker gefunden werden.

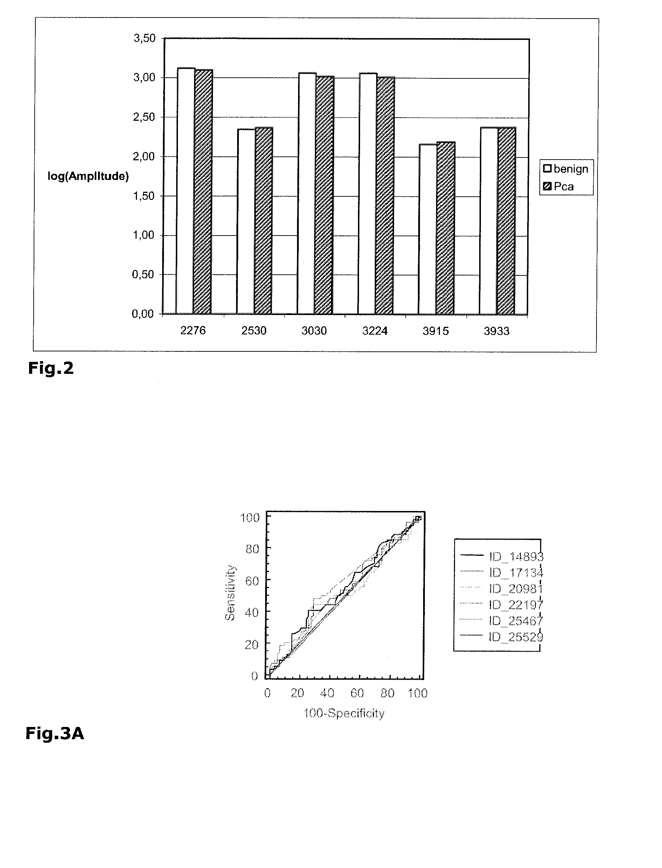

Figur 2 zeigt eine Bestimmung der Amplituden der sechs auf diesem Wege gefundenen Marker. Es zeigt sich, dass zwischen Prostatakarzinompatienten und der Kontrollgruppe keine signifikanten Unterschiede auftreten. Anschließend wurde der diskriminatorische Wert der Biomarker mittels einer ROC-Analyse (Receiver Operator characteristic Curves) untersucht.

Figur 3a zeigt entsprechende Analysen für sechs Marker, die in der WO 01/25791 beschrieben sind und in Urinproben gefunden werden konnten.

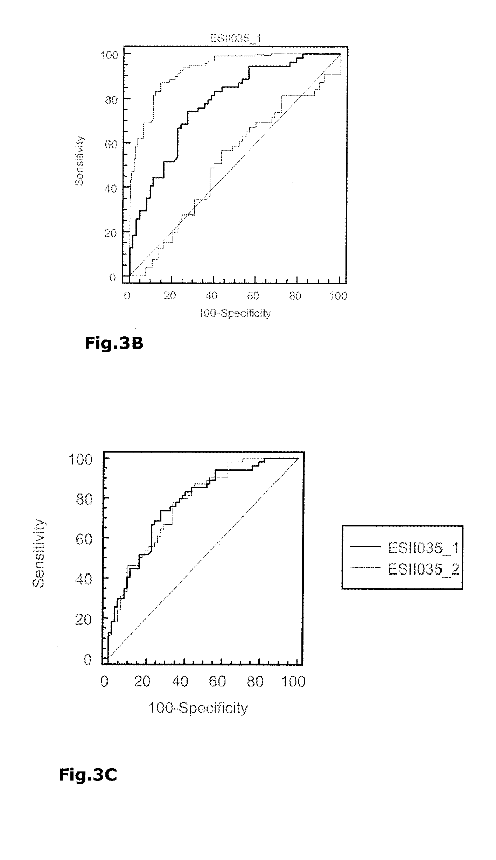

Figur 3b zeigt eine entsprechende ROC-Untersuchung der erfindungsgemäßen Biomarker mit den Nr. 1 bis 78 der Patentanmeldung.

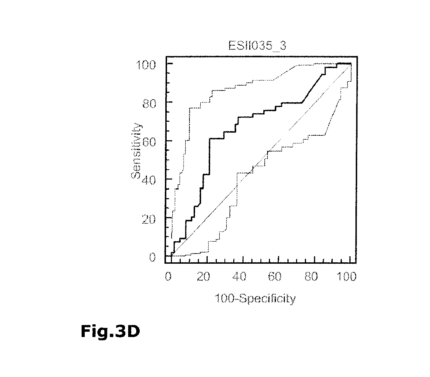

Figur 3c zeigt die ROC-Analyse einer Untergruppe der erfindungsgemäßen Markern (16 Marker) Figur 3d zeigt, dass selbst bei Verwendung von lediglich drei erfindungsgemäßen Biomarkern die Aussagekraft deutlich höher ist als bei der WO 01/25791 liegt, verwendet wurden die Marker 24, 41 und 46.

## Patentansprüche

1. Verfahren zur Diagnose von Prostatakrebs umfassend den Schritt der Bestimmung einer An- oder Abwesenheit mindestens dreier Polypeptidmarkers in einer Probe, wobei der Polypeptidmarker ausgewählt ist aus den Markern 1 bis 44 und 52 bis 78 (Frequenzmarker), oder der Bestimmung der Amplitude mindestens eines Polypeptidmarkers, ausgewählt aus den Markern 45 bis 51 und 79-115 (Amplitudenmarker), die durch folgende Werte für die Molekularmassen und die Migrationszeit charakterisiert sind:
| **Nummer** | **Masse [Da]** | **CE-Zeit** | **Nummer** | **Masse [Da]** | **CE-Zeit** |
|---|---|---|---|---|---|
| 1 | 1579,7 | 26,4 | 27 | 1180,5 | 33,9 |
| 2 | 1991,9 | 17,1 | 28 | 2421,1 | 32,5 |
| 3 | 1955,9 | 24,8 | 29 | 4345,9 | 30,6 |
| 4 | 1140,5 | 21,4 | 30 | 2077,1 | 16,9 |
| 5 | 1677,3 | 7,4 | 31 | 1134,6 | 19,4 |
| 6 | 10753,1 | 13,6 | 32 | 1444,8 | 13,5 |
| 7 | 1412,6 | 22,1 | 33 | 1046,5 | 21,9 |
| 8 | 1636,8 | 27,2 | 34 | 1992,2 | 16,8 |
| 9 | 1946,9 | 28,4 | 35 | 4069,6 | 21,3 |
| 10 | 5887,6 | 25,1 | 36 | 1191,5 | 34,5 |
| 11 | 12407,9 | 22,2 | 37 | 1239,5 | 32,5 |
| 12 | 1186,6 | 17,4 | 38 | 2191,9 | 17,2 |
| 13 | 1240,6 | 23,3 | 39 | 1865,8 | 30,0 |
| 14 | 1326,6 | 24,8 | 40 | 1265,6 | 23,4 |
| 15 | 3802,1 | 30,1 | 41 | 1936,1 | 10,5 |
| 16 | 1749,9 | 19,4 | 42 | 911,3 | 31,9 |
| 17 | 2216,1 | 31,0 | 43 | 1494,7 | 26,4 |
| 18 | 1069,5 | 22,7 | 44 | 1209,6 | 22,5 |
| 19 | 1341,6 | 26,6 | 45 | 1193,3 | 34,2 |
| 20 | 1353,7 | 21,8 | 46 | 1235,4 | 34,3 |
| 21 | 1716,8 | 24,6 | 47 | 1390,5 | 35,0 |
| 22 | 2939,1 | 31,1 | 48 | 2292,1 | 23,7 |
| 23 | 3002,0 | 19,2 | 49 | 2736,3 | 17,1 |
| 24 | 1110,4 | 31,5 | 50 | 3385,5 | 21,0 |
| 25 | 1496,7 | 26,5 | 51 | 3945,2 | 21,4 |
| 26 | 1825,8 | 28,4 | | | |
| | | | | | |
| **Nummer** | **Massen (Da)** | **CE-Zeit [min]** | **Nummer** | **Massen (Da)** | **CE-Zeit [min]** |
|---|---|---|---|---|---|
| 52 | 1636,8 | 27,2 | 66 | 1878,9 | 15,3 |
| 53 | 1412,6 | 22,1 | 67 | 3593,4 | 14,5 |
| 54 | 1579,7 | 26,4 | 68 | 1236,7 | 13,3 |
| 55 | 1390,5 | 35,0 | 69 | 2736,3 | 17,1 |
| 56 | 1196,6 | 15,8 | 70 | 2973,4 | 20,0 |
| 57 | 11041,4 | 16,6 | 71 | 1134,6 | 19,4 |
| 58 | 2971,4 | 17,9 | 72 | 2191,9 | 17,2 |
| 59 | 3136,6 | 20,0 | 73 | 2205,1 | 25,1 |
| 60 | 4409,9 | 14,2 | 74 | 3959,7 | 14,0 |
| 61 | 1494,7 | 26,4 | 75 | 1749,9 | 19,4 |
| 62 | 1833,9 | 24,2 | 76 | 4069,6 | 21,3 |
| 63 | 2752,4 | 14,0 | 77 | 2816,3 | 24,7 |
| 64 | 3515,8 | 15,3 | 78 | 3435,8 | 15,0 |
| 65 | 1082,5 | 17,7 | | | |
| **Nummer** | **Masse [Da]** | **CE-Zeit [min]** |
|---|---|---|
| 79 | 973,26 | 35,44 |
| 80 | 1128,54 | 25,66 |
| 81 | 1173,58 | 37,47 |
| 82 | 1184,6 | 26,43 |
| 83 | 1290,39 | 30,85 |
| 84 | 1338,66 | 23,96 |
| 85 | 1428,45 | 36,73 |
| 86 | 1450,6 | 37,47 |
| 87 | 1460,71 | 19,83 |
| 88 | 1498,46 | 35,31 |
| 89 | 1526,76 | 23,54 |
| 90 | 1588,77 | 30,24 |
| 91 | 1675,77 | 29,23 |
| 92 | 1703,91 | 33,67 |
| 93 | 1808,87 | 23,72 |
| 94 | 1863,96 | 44,01 |
| 95 | 1867,79 | 33,29 |
| 96 | 1925,9 | 23,2 |
| 97 | 2036,97 | 31,53 |
| 98 | 2114,05 | 31,59 |
| 99 | 2196,11 | 33,16 |
| 100 | 2212,06 | 33,36 |
| 101 | 2257,95 | 36,02 |
| 102 | 2544,06 | 26,14 |
| 103 | 2599,21 | 28,05 |
| 104 | 2761,38 | 21,47 |
| 105 | 3334,17 | 31,01 |
| 106 | 3361,41 | 24,32 |
| 107 | 3426,43 | 27,73 |
| 108 | 3765,51 | 20,18 |
| 109 | 3864,56 | 33,82 |
| 110 | 3870,8 | 33,47 |
| 111 | 4252,03 | 28,77 |
| 112 | 6211,93 | 20,27 |
| 113 | 6650,86 | 25,55 |
| 114 | 6820,37 | 21,03 |
| 115 | 16918,24 | 19,8 |
wobei es sich bei der Probe um eine Urinprobe oder Samenflüssigkeitsprobe handelt.

2. Verfahren nach Anspruch 1, **dadurch gekennzeichnet, dass** eine Auswertung der bestimmten An- oder Abwesenheit der Marker 1 bis 44 und 52-78 anhand folgender Referenzwerte erfolgt:
| **Nummer** | **Häufigkeit PCA- Gruppe** | **Häufigkeit Gruppe** | **Kontroll Nummer** | **Häufigkeit PCA-Gruppe** | **Häufigkeit Kontroll-Gruppe** |
|---|---|---|---|---|---|
| 1 | 1 | 0,25 | 23 | 0,25 | 0,78 |
| 2 | 0,88 | 0,22 | 24 | 0,13 | 0,67 |
| 3 | 1 | 0,35 | 25 | 0,13 | 0,67 |
| 4 | 1 | 0,43 | 26 | 0,13 | 0,67 |
| 5 | 0,63 | 0,06 | 27 | 0 | 0,55 |
| 6 | 0,88 | 0,33 | 28 | 0 | 0,55 |
| 7 | 0,75 | 0,22 | 29 | 0 | 0,55 |
| 8 | 0,63 | 0,1 | 30 | 0,13 | 0,69 |
| 9 | 0,75 | 0,22 | 31 | 0,25 | 0,82 |
| 10 | 1 | 0,49 | 32 | 0,13 | 0,71 |
| 11 | 0,75 | 0,24 | 33 | 0 | 0,59 |
| 12 | 0,38 | 0,88 | 34 | 0 | 0,59 |
| 13 | 0,25 | 0,76 | 35 | 0 | 0,59 |
| 14 | 0,38 | 0,88 | 36 | 0 | 0,61 |
| 15 | 0,38 | 0,88 | 37 | 0 | 0,61 |
| 16 | 0,13 | 0,65 | 38 | 0,25 | 0,86 |
| 17 | 0,13 | 0,65 | 39 | 0,13 | 0,75 |
| 18 | 0 | 0,53 | 40 | 0,25 | 0,9 |
| 19 | 0 | 0,53 | 41 | 0 | 0,65 |
| 20 | 0,25 | 0,78 | 42 | 0 | 0,69 |
| 21 | 0,25 | 0,78 | 43 | 0,13 | 0,82 |
| 22 | 0,38 | 0,9 | 44 | 0 | 0,71 |
| **Nummer** | **Massen (Da)** | **CE-Zeit [min]** | **Differenz** | **Faktor PCA-Gruppe** | **Faktor BP-Gruppe** |
|---|---|---|---|---|---|
| 52 | 1636,8 | 27,2 | D:0,63 | 0,63 | 0 |
| 53 | 1412,6 | 22,1 | D:0,57 | 0,75 | 0,18 |
| 54 | 1579,7 | 26,4 | D:0,55 | 1 | 0,45 |
| 55 | 1390,5 | 35,0 | D:0,53 | 0,63 | 0,09 |
| 56 | 1196,6 | 15,8 | D:0,51 | 0,88 | 0,36 |
| 57 | 11041,4 | 16,6 | D:0,51 | 0,88 | 0,36 |
| 58 | 2971,4 | 17,9 | D:0,50 | 0,5 | 0 |
| 59 | 3136,6 | 20,0 | D:-0,50 | 0,5 | 1 |
| 60 | 4409,9 | 14,2 | D:-0,50 | 0,5 | 1 |
| 61 | 1494,7 | 26,4 | D:-0,51 | 0,13 | 0,64 |
| 62 | 1833,9 | 24,2 | D:-0,51 | 0,13 | 0,64 |
| 63 | 2752,4 | 14,0 | D:-0,51 | 0,13 | 0,64 |
| 64 | 3515,8 | 15,3 | D:-0,51 | 0,13 | 0,64 |
| 65 | 1082,5 | 17,7 | D:-0,53 | 0,38 | 0,91 |
| 66 | 1878,9 | 15,3 | D:-0,53 | 0,38 | 0,91 |
| 67 | 3593,4 | 14,5 | D:-0,53 | 0,38 | 0,91 |
| 68 | 1236,7 | 13,3 | D:-0,55 | 0 | 0,55 |
| 69 | 2736,3 | 17,1 | D:-0,55 | 0 | 0,55 |
| 70 | 2973,4 | 20,0 | D:-0,55 | 0 | 0,55 |
| 71 | 1134,6 | 19,4 | D:-0,57 | 0,25 | 0,82 |
| 72 | 2191,9 | 17,2 | D:-0,57 | 0,25 | 0,82 |
| 73 | 2205,1 | 25,1 | D:-0,57 | 0,25 | 0,82 |
| 74 | 3959,7 | 14,0 | D:-0,57 | 0,25 | 0,82 |
| 75 | 1749,9 | 19,4 | D:-0,60 | 0,13 | 0,73 |
| 76 | 4069,6 | 21,3 | D:-0,64 | 0 | 0,64 |
| 77 | 2816,3 | 24,7 | D:-0,73 | 0 | 0,73 |
| 78 | 3435,8 | 15,0 | D:-0,82 | 0 | 0,82 |

3. Verfahren nach Anspruch 1, **dadurch gekennzeichnet, dass** eine Auswertung der Amplitude der Marker 45 bis 51 und 79 bis 115 anhand folgender Referenzwerte erfolgt:
| **Nummer** | **Masse [Da]** | **CE-Zeit [min]** | **Mittlere Amplitude in PCA-Proben [ppm]** | **Mittlere Amplitude in Kontroll-Proben** [ppm] |
|---|---|---|---|---|
| 45 | 1193,3 | 34,2 | 24000 | 11000 |
| 46 | 1235,4 | 34,3 | 160 | 370 |
| 47 | 1390,5 | 35,0 | 22000 | 12000 |
| 48 | 2292,1 | 23,7 | 2600 | 1600 |
| 49 | 2736,3 | 17,1 | 120 | 350 |
| 50 | 3385,5 | 21,0 | 2600 | 1700 |
| 51 | 3945,2 | 21,4 | 260 | 120 |
| **Nummer** | **Masse [Da]** | **CE-Zeit** | **Mittl. Amplitude PCa-Proben ppm** | **Mittl. Amplitude in Kontroll-Proben ppm** |
|---|---|---|---|---|
| 79 | 973,26 | 35,44 | 112,27 | 94,56 |
| 80 | 1128,54 | 25,66 | 104,1 | 135,92 |
| 81 | 1173,58 | 37,47 | 163,08 | 174,37 |
| 82 | 1184,6 | 26,43 | 61,33 | 53,82 |
| 83 | 1290,39 | 30,85 | 425,14 | 360,22 |
| 84 | 1338,66 | 23,96 | 116,85 | 223,77 |
| 85 | 1428,45 | 36,73 | 2325,09 | 1972,91 |
| 86 | 1450,6 | 37,47 | 348,86 | 350,46 |
| 87 | 1460,71 | 19,83 | 204,2 | 216,45 |
| 88 | 1498,46 | 35,31 | 115,26 | 94,66 |
| 89 | 1526,76 | 23,54 | 94,2 | 155,52 |
| 90 | 1588,77 | 30,24 | 504,83 | 565,2 |
| 91 | 1675,77 | 29,23 | 104,37 | 83,95 |
| 92 | 1703,91 | 33,67 | 326,26 | 495,27 |
| 93 | 1808,87 | 23,72 | 189,86 | 198,11 |
| 94 | 1863,96 | 44,01 | 989,35 | 1411,72 |
| 95 | 1867,79 | 33,29 | 117,97 | 108,75 |
| 96 | 1925,9 | 23,2 | 148,34 | 145,13 |
| 97 | 2036,97 | 31,53 | 74,45 | 77,29 |
| 98 | 2114,05 | 31,59 | 135,48 | 121,91 |
| 99 | 2196,11 | 33,16 | 479,36 | 390,89 |
| 100 | 2212,06 | 33,36 | 336,96 | 397,62 |
| 101 | 2257,95 | 36,02 | 365,38 | 567,62 |
| 102 | 2544,06 | 26,14 | 77,74 | 121,23 |
| 103 | 2599,21 | 28,05 | 797,34 | 915,08 |
| 104 | 2761,38 | 21,47 | 744,83 | 616,6 |
| 105 | 3334,17 | 31,01 | 105,87 | 127,64 |
| 106 | 3361,41 | 24,32 | 143,28 | 108,31 |
| 107 | 3426,43 | 27,73 | 149,46 | 116,27 |
| 108 | 3765,51 | 20,18 | 375,75 | 309,53 |
| 109 | 3864,56 | 33,82 | 271,44 | 162,02 |
| 110 | 3870,8 | 33,47 | 71,63 | 78,31 |
| 111 | 4252,03 | 28,77 | 388,07 | 447,93 |
| 112 | 6211,93 | 20,27 | 492,21 | 400,75 |
| 113 | 6650,86 | 25,55 | 233,38 | 232,51 |
| 114 | 6820,37 | 21,03 | 258,88 | 227,62 |
| 115 | 16918,24 | 19,8 | 605,64 | 324, 52 |

4. Verfahren nach Anspruch 1, wobei mindestens vier oder mindestens fünf Polypeptidmarker verwendet werden, wie sie in Anspruch 1 definiert sind.

5. Verfahren nach einem der Ansprüche 1 bis 3, wobei mindestens zehn oder alle Polypeptidmarker verwendet werden, wie sie in Anspruch 1 definiert sind.

6. Verfahren zur Differenzialdiagnose zwischen Prostatakrebs (PCA) und benigner Prostatahyperplasie (BPH) umfassend den Schritt der Bestimmung einer An- oder Abwesenheit von mindestens drei Polypeptidmarkern in einer Probe, wobei der Polypeptidmarker ausgewählt ist aus den Markern 52 bis 78, die durch folgende Werte für die Molekularmassen und die Migrationszeit charakterisiert sind:
| **Nummer** | **Massen (Da)** | **CE-Zeit [min]** | **Nummer** | **Massen (Da)** | **CE-Zeit [min]** |
|---|---|---|---|---|---|
| 52 | 1636,8 | 27,2 | 66 | 1878,9 | 15,3 |
| 53 | 1412,6 | 22,1 | 67 | 3593,4 | 14,5 |
| 54 | 1579,7 | 26,4 | 68 | 1236,7 | 13,3 |
| 55 | 1390,5 | 35,0 | 69 | 2736,3 | 17,1 |
| 56 | 1196,6 | 15,8 | 70 | 2973,4 | 20,0 |
| 57 | 11041,4 | 16,6 | 71 | 1134,6 | 19,4 |
| 58 | 2971,4 | 17,9 | 72 | 2191,9 | 17,2 |
| 59 | 3136,6 | 20,0 | 73 | 2205,1 | 25,1 |
| 60 | 4409,9 | 14,2 | 74 | 3959,7 | 14,0 |
| 61 | 1494,7 | 26,4 | 75 | 1749,9 | 19,4 |
| 62 | 1833,9 | 24,2 | 76 | 4069,6 | 21,3 |
| 63 | 2752,4 | 14,0 | 77 | 2816,3 | 24,7 |
| 64 | 3515,8 | 15,3 | 78 | 3435,8 | 15,0 |
| 65 | 1082,5 | 17,7 | | | |
wobei es sich bei der Probe um eine Urinprobe oder Samenflüssigkeitsprobe handelt.

7. Verfahren nach Anspruch 6, **dadurch gekennzeichnet, dass** eine Auswertung der bestimmten An- oder Abwesenheit anhand folgender Referenzwerte erfolgt:
| **Nummer** | **Häufigkeit PCA-Gruppe** | **Häufigkeit BP-Gruppe** | **Nummer** | **Häufigkeit PCA-Gruppe** | **Häufigkeit BP-Gruppe** |
|---|---|---|---|---|---|
| 52 | 0,63 | 0 | 66 | 0,38 | 0,91 |
| 53 | 0,75 | 0,18 | 67 | 0,38 | 0,91 |
| 54 | 1 | 0,45 | 68 | 0 | 0,55 |
| 55 | 0,63 | 0,09 | 69 | 0 | 0,55 |
| 56 | 0,88 | 0,36 | 70 | 0 | 0,55 |
| 57 | 0,88 | 0,36 | 71 | 0,25 | 0,82 |
| 58 | 0,5 | 0 | 72 | 0,25 | 0,82 |
| 59 | 0,5 | 1 | 73 | 0,25 | 0,82 |
| 60 | 0,5 | 1 | 74 | 0,25 | 0,82 |
| 61 | 0,13 | 0,64 | 75 | 0,13 | 0,73 |
| 62 | 0,13 | 0,64 | 76 | 0 | 0,64 |
| 63 | 0,13 | 0,64 | 77 | 0 | 0,73 |
| 64 | 0,13 | 0,64 | 78 | 0 | 0,82 |
| 65 | 0,38 | 0,91 | | | |

8. Verfahren nach Anspruch 6 wobei mindestens drei oder mindestens vier oder mindestens fünf oder mindestens zehn oder alle Polypeptidmarker verwendet werden, wie sie in Anspruch 6 definiert sind.

9. Verfahren nach einem der Ansprüche 1 bis 8, wobei Kapillarelektrophorese, HPLC, Gasphasenionenspektrometrie und/oder Massenspektrometrie zum Nachweis der An- oder Abwesenheit des/der Polypeptidmarker verwendet wird.

10. Verfahren nach einem der Ansprüche 1 bis 9, wobei vor der Messung der Molekularmasse der Polypeptidmarker eine Kapillarelektrophorese durchgeführt wird.

11. Verfahren nach einem der Ansprüche 1 bis 10, wobei Massenspektrometrie zum Nachweis der An- oder Abwesenheit des/der Polypeptidmarker verwendet wird.

12. Verwendung mindestens eines Polypeptidmarkers ausgewählt aus den Markern Nr. 1 bis 115, der durch die folgenden Werte für die Molekularmassen und die Migrationszeit charakterisiert ist
| **Nummer** | **Masse [Da]** | **CE-Zeit** | **Nummer** | **Masse [Da]** | **CE-Zeit** |
|---|---|---|---|---|---|
| 1 | 1579,7 | 26,4 | 27 | 1180,5 | 33,9 |
| 2 | 1991,9 | 17,1 | 28 | 2421,1 | 32,5 |
| 3 | 1955,9 | 24,8 | 29 | 4345,9 | 30,6 |
| 4 | 1140,5 | 21,4 | 30 | 2077,1 | 16,9 |
| 5 | 1677,3 | 7,4 | 31 | 1134,6 | 19,4 |
| 6 | 10753,1 | 13,6 | 32 | 1444,8 | 13,5 |
| 7 | 1412,6 | 22,1 | 33 | 1046,5 | 21,9 |
| 8 | 1636,8 | 27,2 | 34 | 1992,2 | 16,8 |
| 9 | 1946,9 | 28,4 | 35 | 4069,6 | 21,3 |
| 10 | 5887,6 | 25,1 | 36 | 1191,5 | 34,5 |
| 11 | 12407,9 | 22,2 | 37 | 1239,5 | 32,5 |
| 12 | 1186,6 | 17,4 | 38 | 2191,9 | 17,2 |
| 13 | 1240,6 | 23,3 | 39 | 1865,8 | 30,0 |
| 14 | 1326,6 | 24,8 | 40 | 1265,6 | 23,4 |
| 15 | 3802,1 | 30,1 | 41 | 1936,1 | 10,5 |
| 16 | 1749,9 | 19,4 | 42 | 911,3 | 31,9 |
| 17 | 2216,1 | 31,0 | 43 | 1494,7 | 26,4 |
| 18 | 1069,5 | 22,7 | 44 | 1209,6 | 22,5 |
| 19 | 1341,6 | 26,6 | 45 | 1193,3 | 34,2 |
| 20 | 1353,7 | 21,8 | 46 | 1235,4 | 34,3 |
| 21 | 1716,8 | 24,6 | 47 | 1390,5 | 35,0 |
| 22 | 2939,1 | 31,1 | 48 | 2292,1 | 23,7 |
| 23 | 3002,0 | 19,2 | 49 | 2736,3 | 17,1 |
| 24 | 1110,4 | 31,5 | 50 | 3385,5 | 21,0 |
| 25 | 1496,7 | 26,5 | 51 | 3945,2 | 21,4 |
| 26 | 1825,8 | 28,4 | | | |
| | | | | | |
| **Nummer** | **Massen (Da)** | **CE-Zeit [min]** | **Nummer** | **Massen (Da)** | **CE-Zeit [min]** |
|---|---|---|---|---|---|
| 52 | 1636,8 | 27,2 | 66 | 1878,9 | 15,3 |
| 53 | 1412,6 | 22,1 | 67 | 3593,4 | 14,5 |
| 54 | 1579,7 | 26,4 | 68 | 1236,7 | 13,3 |
| 55 | 1390,5 | 35,0 | 69 | 2736,3 | 17,1 |
| 56 | 1196,6 | 15,8 | 70 | 2973,4 | 20,0 |
| 57 | 11041,4 | 16,6 | 71 | 1134,6 | 19,4 |
| 58 | 2971,4 | 17,9 | 72 | 2191,9 | 17,2 |
| 59 | 3136,6 | 20,0 | 73 | 2205,1 | 25,1 |
| 60 | 4409,9 | 14,2 | 74 | 3959,7 | 14,0 |
| 61 | 1494,7 | 26,4 | 75 | 1749,9 | 19,4 |
| 62 | 1833,9 | 24,2 | 76 | 4069,6 | 21,3 |
| 63 | 2752,4 | 14,0 | 77 | 2816,3 | 24,7 |
| 64 | 3515,8 | 15,3 | 78 | 3435,8 | 15,0 |
| 65 | 1082,5 | 17,7 | | | |
| **Nummer** | **Masse [Da]** | **CE-Zeit [min]** |
|---|---|---|
| 79 | 973,26 | 35,44 |
| 80 | 1128,54 | 25,66 |
| 81 | 1173,58 | 37,47 |
| 82 | 1184,6 | 26,43 |
| 83 | 1290,39 | 30,85 |
| 84 | 1338,66 | 23,96 |
| 85 | 1428,45 | 36,73 |
| 86 | 1450,6 | 37,47 |
| 87 | 1460,71 | 19,83 |
| 88 | 1498,46 | 35,31 |
| 89 | 1526,76 | 23,54 |
| 90 | 1588,77 | 30,24 |
| 91 | 1675,77 | 29,23 |
| 92 | 1703,91 | 33,67 |
| 93 | 1808,87 | 23,72 |
| 94 | 1863,96 | 44,01 |
| 95 | 1867,79 | 33,29 |
| 96 | 1925,9 | 23,2 |
| 97 | 2036,97 | 31,53 |
| 98 | 2114,05 | 31,59 |
| 99 | 2196,11 | 33,16 |
| 100 | 2212,06 | 33,36 |
| 101 | 2257,95 | 36,02 |
| 102 | 2544,06 | 26,14 |
| 103 | 2599,21 | 28,05 |
| 104 | 2761,38 | 21,47 |
| 105 | 3334,17 | 31,01 |
| 106 | 3361,41 | 24,32 |
| 107 | 3426,43 | 27,73 |
| 108 | 3765,51 | 20,18 |
| 109 | 3864,56 | 33,82 |
| 110 | 3870,8 | 33,47 |
| 111 | 4252,03 | 28,77 |
| 112 | 6211,93 | 20,27 |
| 113 | 6650,86 | 25,55 |
| 114 | 6820,37 | 21,03 |
| 115 | 16918,24 | 19,8 |
zur Diagnose von Prostatakrebs.

13. Verwendung mindestens eines Polypeptidmarkers ausgewählt aus den Markern Nr. 52 bis 78, der durch die folgenden Werte für die Molekularmassen und die Migrationszeit charakterisiert ist
| **Nummer** | **Massen (Da)** | **CE-Zeit [min]** | **Nummer** | **Massen (Da)** | **CE-Zeit [min]** |
|---|---|---|---|---|---|
| 52 | 1636,8 | 27,2 | 66 | 1878,9 | 15,3 |
| 53 | 1412,6 | 22,1 | 67 | 3593,4 | 14,5 |
| 54 | 1579,7 | 26,4 | 68 | 1236,7 | 13,3 |
| 55 | 1390,5 | 35,0 | 69 | 2736,3 | 17,1 |
| 56 | 1196,6 | 15,8 | 70 | 2973,4 | 20,0 |
| 57 | 11041,4 | 16,6 | 71 | 1134,6 | 19,4 |
| 58 | 2971,4 | 17,9 | 72 | 2191,9 | 17,2 |
| 59 | 3136,6 | 20,0 | 73 | 2205,1 | 25,1 |
| 60 | 4409,9 | 14,2 | 74 | 3959,7 | 14,0 |
| 61 | 1494,7 | 26,4 | 75 | 1749,9 | 19,4 |
| 62 | 1833,9 | 24,2 | 76 | 4069,6 | 21,3 |
| 63 | 2752,4 | 14,0 | 77 | 2816,3 | 24,7 |
| 64 | 3515,8 | 15,3 | 78 | 3435,8 | 15,0 |
| 65 | 1082,5 | 17,7 | | | |
zur Differentialdiagnose zwischen Prostatakrebs und benigner Prostatahyperplasie (BPH).

14. Verwendung nach einem der Ansprüche 12 oder 13, wobei zur Diagnose mindestens drei Marker verwendet werden.

15. Polypeptid mit der Sequenz: REVQSKIGYGRQIIS.


SEQUENCE LISTING

<110>  mosaiques diagnostics and therapeutics AG

<120>  Polypeptidmarker zur Diagnose von Prostatakrebs

<130>  060867wo

<140>  PCT/EP2006/061374
<141>  2006-04-04

<150>  EP05102741.5
<151>  2005-04-07

<160>  4

<170>  PatentIn version 3.3

<210>  1
<211>  15
<212>  PRT
<213>  Artificial

<220>
<223>  Synthetisches Peptid

<400>  1

Arg Glu Val Gln Ser Lys Ile Gly Tyr Gly Arg Gln Ile Ile Ser
1               5                   10                  15


<210>  2
<211>  24
<212>  PRT
<213>  Artificial

<220>
<223>  Synthetisches Peptid

<400>  2

Glu Leu Met Thr Gly Glu Leu Pro Tyr Ser His Ile Asn Asn Arg Asp
1               5                   10                  15


Gln Ile Ile Phe Met Val Gly Arg
            20


<210>  3
<211>  21
<212>  PRT
<213>  Artificial

<220>
<223>  Synthetisches Peptid

<400>  3

Thr Gly Ser Leu Pro Tyr Ser His Ile Gly Ser Arg Asp Gln Ile Ile
1               5                   10                  15

Phe Met Val Gly Arg
            20

<210>  4
<211>  18
<212>  PRT
<213>  Artificial

<220>
<223>  Synthetisches Peptid

<400>  4

Gly Ile Val Leu Tyr Glu Leu Met Thr Gly Glu Leu Pro Tyr Ser His
1               5                   10                  15

Ile Asn

- 3 -